# EUROPEAN PATENT APPLICATION

(11) **EP 4 837 912 A1**
(43) Date of publication of application: **11.11.2026**
(21) Application number: 25736398.6
(22) Date of filing: 03.01.2025
(51) Int. Cl.: C07D 498/04, A61K 31/437, A61P 29/00, A61P 37/00

(54) **HETEROCYCLIC COMPOUND AND PHARMACEUTICAL COMPOSITION THEREOF**

(30) Priority: 05.01.2024 CN 202410019637
(71) Applicant: Prime Gene Therapeutics Co., Ltd., Beijing 100176 (CN)
(72) Inventor: HU, Wei, Beijing 100176 (CN); ZHU, Li, Beijing 100176 (CN); DAI, Liguang, Beijing 100176 (CN); YANG, Yanqing, Beijing 100176 (CN); ZHANG, Hui, Beijing 100176 (CN); DU, Daniel Yunlong, Beijing 100176 (CN)
(74) Representative: Winter, Brandl - Partnerschaft mbB
(86) International application number: PCT/CN2025/070537
(87) International publication number: WO 2025/146160

(57) **Abstract**

The present application relates to a heterocyclic compound and a pharmaceutical composition thereof. The compound has the structure of formula I and is a potent NLRP3 inhibitor, which can be used for treating various NLRP3-mediated diseases.

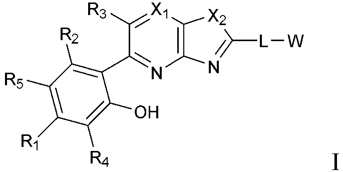

## Description

### FIELD OF THE INVENTION

The present application provides a heterocyclic compound, a pharmaceutical composition and uses thereof. The heterocyclic compound is an NLRP3 inhibitor which can be used for modulating various NLRP3-mediated diseases.

### BACKGROUND OF THE INVENTION

Inflammatory response is a common physiological and pathological activity in the body, and inflammasomes play an important regulatory role in this response. Currently, five main types of inflammasomes have been discovered, namely the NLRP1 inflammasome, the NLRP3 inflammasome, the NLRC4 inflammasome, the IPAF inflammasome, and the AIM2 inflammasome. Among them, the NLRP3 inflammasome is the most extensively and thoroughly studied.

NLRP3 is a protein of the NLR (NOD-like receptor) family and is one of the core members of the inflammasome. It is composed of multiple proteins, including NLRP3, ASC (apoptosis-associated speck-like protein containing a CARD), and pro-caspase-1. NLRP3 is the sensor of the inflammasome, which interacts with other proteins through its NACHT domain and LRR domain to form the activated inflammasome. ASC is an adapter protein that interacts with NLRP3 through its CARD (caspase activation and recruitment domain) structure, promoting the formation of the inflammasome. Pro-caspase-1 is a zymogen, which is activated into mature caspase-1 upon inflammasome formation, and further regulates intracellular inflammatory responses.

The activation process of NLRP3 involves multiple signaling pathways. Generally, the activation of NLRP3 requires two signals: the first signal is through inducing transcription and protein expression of NLRP3, for example, through activation of the Tol-like receptor (TLR) signaling pathway; the second signal typically originates from intracellular damage signals or other stimuli, such as intracellular oxidative stress, potassium ion efflux, or mitochondrial dysfunction. When both signals are present simultaneously, NLRP3 is activated and forms the inflammasome, thereby promoting the occurrence of inflammatory responses.

NLRP3 plays an important regulatory role in inflammatory processes. When the body suffers injury, infection, or other stimuli, NLRP3 is activated and forms the inflammasome. The NLRP3 inflammasome participates in the onset and development of diseases primarily by promoting the massive release of downstream inflammatory cytokines to induce acute and chronic inflammatory responses in the body. The relevant downstream cytokines thereof can participate in the onset and development of immune-related diseases or parasitic diseases by modulating the function of immune cells. For example, IL-1β can promote the aggregation of Th2 cells and the differentiation of Th17 cells; IL-18 can simultaneously enhance the immune response involving both Th1 and Th2 cells; and IL-33 can mediate the response of Th2 cells.

NLRP3 is one of the core members of the inflammasome, the activation of which and the formation of inflammasome play crucial regulatory roles in inflammatory responses. The activation of NLRP3 is closely associated with the onset and development of various diseases, including inflammatory diseases, autoimmune diseases, and tumors, etc. Hyperactivation of NLRP3 is associated with the development of many inflammatory diseases such as gout, obesity, diabetes, and ACS, etc. Therefore, as the core of the inflammatory response, the NLRP3 inflammasome may provide new approaches for the treatment of various inflammatory diseases.

### SUMMARY OF THE INVENTION

The present application provides a novel heterocyclic compound, which is a potent NLRP3 inhibitor and can be used for treating various NLRP3- mediated diseases.

The present application relates to a compound of Formula I 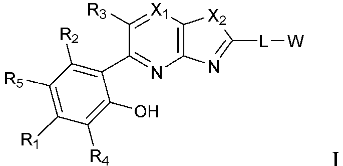 or a pharmaceutically acceptable salt, hydrate, solvate, active metabolite, polymorph, isotopically labeled compound, isomer, or prodrug thereof.

In some particularly preferred embodiments, the compound of Formula I of the present invention is selected from:

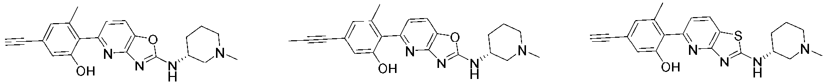

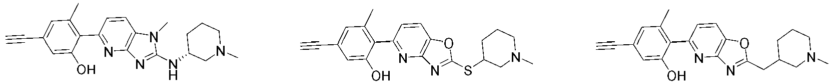

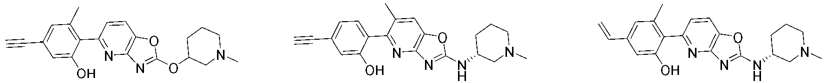

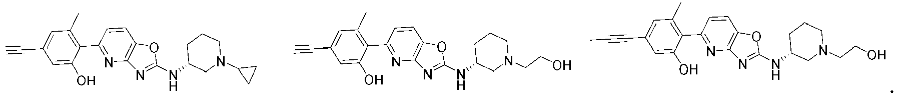

Another aspect of the present invention provides the use of the compound of Formula I, or a pharmaceutically acceptable salt, hydrate, solvate, active metabolite, polymorph, isotopically labeled compound, isomer, or prodrug thereof, in the manufacture of a pharmaceutical for treating NLRP3-mediated diseases.

### DETAILED DESCRIPTION OF THE INVENTION

The present application will be described in further detail below through examples. The features and advantages of the present application will become clearer from these descriptions.

The term "exemplary" used herein specifically means "served as an example, embodiment, or illustrative". Any example illustrated herein as "exemplary" need not be construed as superior to or better than any other example.

In addition, the technical features involved in various embodiments of the present application described below may be combined with each other as long as they do not conflict.

### Definitions

The chemical nomenclature schemes and structural formulae used herein are modified forms of the I.U.P.A.C. nomenclature system, using the ACD/Name 9.07 version software program and/or the ChemDraw Professional 17.0.0.206 version naming program (CambridgeSoft). For complex chemical names used herein, substituents are usually named before the group to which they are attached. For example, cyclopropylethyl comprises an ethyl backbone with a cyclopropyl substituent. Unless described below, all bonds are identified in the chemical structural formulae herein, but it is assumed that all bonds on certain carbon atoms are bonded to sufficient hydrogen atoms to complete the valence.

Unless the context requires otherwise, throughout the specification and claims, the term "comprise" and variations thereof (e.g., "comprises" and "comprising") are to be interpreted in an open, inclusive sense, i.e., as "including, but not limited to."

In this description, any concentration range, percentage range, ratio range, or integer range should be understood to include the value of any integer within the stated range and, where appropriate, fractions thereof (e.g., tenths and hundredths of an integer), unless otherwise indicated. As used herein, the terms "about" and "approximately" mean +20%, +10%, +5%, or +1% of the indicated range, value, or structure, unless otherwise indicated. It should be understood that the terms "a" and "an" as used herein refer to "one or more" of the enumerated components. The use of alternatives (e.g., "or") should be understood to mean either or both of the alternatives, or any combination thereof.

References throughout the description to "one embodiment" or "an embodiment" mean that a particular feature, structure, or characteristic described in connection with the embodiment is included in at least one embodiment of the present invention. Thus, the appearances of the phrases "in one embodiment" or "in an embodiment" in various places throughout this description are not necessarily all referring to the same embodiment. Furthermore, the particular features, structures, or characteristics may be combined in any suitable manner in one or more embodiments.

Unless otherwise defined, all technical and scientific terms used herein have the same meaning as commonly understood by one of ordinary skill in the art to which this disclosure belongs. As used in this specification and the claims, the singular forms "a," "an," and "the" include plural referents unless the context clearly dictates otherwise.

"Cyano" refers to a -CN group.

"Hydroxy" or "hydroxyl" refers to an -OH group.

"Oxo" refers to the =O substituent.

"Mercapto" refers to the -SH substituent.

"Thio" refers to the =S substituent.

"Alkyl" refers to a saturated straight or branched chain hydrocarbyl group consisting solely of carbon and hydrogen atoms, having 1 to 12 carbon atoms (C₁-C₁₂ alkyl), 1 to 8 carbon atoms (C₁-C₈ alkyl), or 1 to 6 carbon atoms (C₁-C₆ alkyl), or any value within these ranges (e.g., C₄-C₆ alkyl, etc.), and which is attached to the rest of the molecule by a single bond, for example, methyl, ethyl, n-propyl, 1-methylethyl (isopropyl), n-butyl, n-pentyl, 1,1-dimethylethyl (tert-butyl), 3-methylhexyl, 2-methylhexyl, etc. The mentioned number of carbon atoms relates to the carbon atoms of backbone and branch chain, but does not include carbon atoms belonging to any substituents. Unless otherwise specifically stated in the specification, the alkyl group is optionally substituted.

"Alkenyl" refers to an unsaturated straight or branched chain hydrocarbyl group consisting solely of carbon and hydrogen atoms, containing one or more carbon-carbon double bonds, having 2 to 12 carbon atoms (C₂-C₁₂ alkenyl), 2 to 8 carbon atoms (C₂-C₈ alkenyl), or 2 to 6 carbon atoms (C₂-C₆ alkenyl), or any value within these ranges, and which is attached to the rest of the molecule by a single bond, for example, vinyl, prop-1-enyl, but-1-enyl, pent-1-enyl, penta-1,4-dienyl, etc. The mentioned number of carbon atoms relates to carbon atoms of backbone and branch chain, but does not include carbon atoms belonging to any substituents. Unless otherwise specifically stated in this specification, the alkenyl group is optionally substituted.

The term "alkynyl" refers to an unsaturated straight or branched chain hydrocarbyl group having 2 to 12 carbon atoms (C₂-C₁₂ alkynyl), 2 to 9 carbon atoms (C₂-C₉ alkynyl), or 2 to 6 carbon atoms (C₂-C₆ alkynyl), or any value within these ranges, and having at least one carbon-carbon triple bond. Examples of alkynyl groups may be selected from the group consisting of ethynyl, propargyl, but-1-ynyl, but-2-ynyl, and the like. The mentioned number of carbon atoms relates to carbon atoms of backbone and branch chain, but does not include carbon atoms belonging to any substituents. Unless otherwise specifically stated in this specification, the alkynyl group is optionally substituted.

"Alkoxy" refers to a group of the formula -OR, wherein R is alkyl as defined above, which may contain 1 to 12 carbon atoms (C₁-C₁₂ alkoxy), 1 to 8 carbon atoms (C₁-C₈ alkoxy), or 1 to 6 carbon atoms (C₁-C₆ alkoxy), or any value within these ranges. Unless otherwise specifically stated in the specification, the alkoxy group is optionally substituted.

"Carbocyclic" or "carbocycle" refers to a ring system in which each of the ring atoms is carbon.

"Cyclic hydrocarbyl group" refers to a non-aromatic monocyclic or polycyclic carbocyclic group consisting solely of carbon and hydrogen atoms, which may include fused or bridged ring systems, having 3 to 15 ring carbon atoms (C₃-C₁₅ cyclic hydrocarbyl group), 3 to 10 ring carbon atoms (C₃-C₁₀ cyclic hydrocarbyl group), or 3 to 8 ring carbon atoms (C₃-C₈ cyclic hydrocarbyl group), or any value within these ranges, for example, 3 to 4 carbon atoms (C₃-C₄ cyclic hydrocarbyl group), and which is saturated or partially unsaturated and is attached to the rest of the molecule by a single bond. Monocyclic groups include, for example, cyclopropyl, cyclobutyl, cyclopentyl, cyclohexyl, cycloheptyl, and cyclooctyl. Polycyclic groups include, for example, adamantyl, norbornyl, decalinyl, 7,7-dimethyl-bicyclo[2.2.1]heptyl, and the like. Unless otherwise specifically stated in the specification, the cyclic hydrocarbyl group group is optionally substituted.

"Alkyl cyclic hydrocarbyl group" refers to a group of the formula -RₐR_{b}, wherein Rₐ is a cyclic hydrocarbyl group and R_{b} is alkyl as defined above. Unless otherwise specifically stated in the specification, the alkyl cyclic hydrocarbyl group is optionally substituted.

"Fused" refers to any ring structure described herein that is fused to another ring structure.

"Halogen" refers to bromine, chlorine, fluorine, or iodine.

"Hydrogen" refers to the various isotopes of hydrogen, including protium, deuterium, and tritium; in particular, when hydrogen appears alone, it may be any one of "protium", "deuterium", and "tritium"; when hydrogen appears together with "deuterium" and/or "tritium", it sometimes refers to "protium".

"Haloalkyl" refers to alkyl as defined above substituted with one or more halogen groups as defined above, for example, trifluoromethyl, difluoromethyl, trichloromethyl, 2,2,2-trifluoroethyl, 1,2-difluoroethyl, 3-bromo-2-fluoropropyl, 1,2-dibromoethyl, and the like. Unless otherwise specifically stated in the specification, the haloalkyl group is optionally substituted.

"Halo cyclic hydrocarbyl group" refers to a cyclic hydrocarbyl group as defined above substituted with one or more halogen groups as defined above, for example, trifluoromethyl, difluoromethyl, trichloromethyl, 2,2,2-trifluoroethyl, 1,2-difluoroethyl, 3-bromo-2-fluoropropyl, 1,2-dibromoethyl, and the like. Unless otherwise specifically stated in the specification, the halo cyclic hydrocarbyl group is optionally substituted.

"Haloalkyl cyclic hydrocarbyl group" refers to a group of the formula -RₐR_{b}, wherein Rₐ is cyclic hydrocarbyl group and R_{b} is haloalkyl as defined above. Unless otherwise specifically stated in the specification, the haloalkyl cyclic hydrocarbyl group is optionally substituted.

"Hydroxyalkyl" refers to alkyl as defined above substituted with one or more hydroxy groups. Hydroxyalkyl is attached at the main chain through the alkyl carbon atom. Unless otherwise specifically stated in the specification, the hydroxyalkyl group is optionally substituted.

"Heterocyclyl" refers to a 3- to 18-membered (for example, 3- to 10-membered or 3- to 8-membered) non-aromatic ring group having 1 to 10 ring carbon atoms (e.g., 2 to 10) and 1 to 6 ring heteroatoms selected from the group consisting of nitrogen, oxygen, and sulfur. Unless otherwise specifically stated in this specification, the heterocyclyl group is partially or fully saturated and is a monocyclic, bicyclic, tricyclic, or tetracyclic ring system, which may include fused, spiro, and/or bridged ring systems. Nitrogen, carbon, and sulfur atoms in the heterocyclyl group may be optionally oxidized, and nitrogen atoms may be optionally quaternized. Examples of such heterocyclyl groups include, but are not limited to, dioxolanyl, thienyl[1,3]dithianyl, decahydroisoquinolinyl, furanonyl, imidazolinyl, imidazolidinyl, isothiazolidinyl, isoxazolidinyl, morpholinyl, octahydroindolyl, octahydroisoindolyl, hexahydro-1H-pyrrolizine, 2-oxopiperazinyl, 2-oxopiperidinyl, 2-oxopyrrolidinyl, oxazolidinyl, oxiranyl, piperidinyl, piperazinyl, 4-piperidonyl, azetidinyl, pyrrolidinyl, pyrazolidinyl, quinuclidinyl, thiazolidinyl, tetrahydrofuranyl, trithianyl, tetrahydropyranyl, thiomorpholinyl, thiamorpholinyl, 1-oxo-thiomorpholinyl, and 1,1-dioxo-thiomorpholinyl. Unless otherwise specifically stated in the specification, the heterocyclyl group is optionally substituted.

As used herein, the term "substituted" means any of the above-mentioned groups (e.g., alkyl, alkenyl, alkylene, alkylcarbonyl, alkoxy, alkoxyalkyl, aminoalkyl, aryl, cyanoalkyl, cyclic hydrocarbyl group, haloalkyl, heterocyclyl, heterocyclenyl, heterocyclylalkyl, heteroaryl, heteroarylalkyl, and/or hydroxyalkyl) in which at least one hydrogen atom (e.g., 1, 2, 3, or all hydrogen atoms) is replaced by a bond to a non-hydrogen substituent. Examples of non-hydrogen substituents include, but are not limited to: amino, carboxyl, cyano, hydroxy, halogen, nitro, oxo, thiol, thio, alkyl, alkenyl, alkylcarbonyl, alkoxy, aryl, cyanoalkyl, cyclic hydrocarbyl group, haloalkyl, heterocyclyl, heterocyclylalkyl, heteroaryl, heteroarylalkyl, and/or hydroxyalkyl substituents, which may also be each optionally substituted with one or more of the above substituents.

The term "effective amount" or "therapeutically effective amount" refers to an amount of a compound described herein sufficient to achieve the intended application, including but not limited to disease treatment as defined below. The therapeutically effective amount may vary depending on the intended therapeutic application (in vivo), or the individual and disease condition being treated, e.g., the weight and age of the individual, the severity of the disease condition, the manner of administration, etc., which can be readily determined by one of ordinary skill in the art. This term also applies to a dose that induces a specific response in target cells, e.g., reduction of platelet adhesion and/or cell migration. The specific dosage will vary depending on the specific compound selected, the administration regimen followed, whether it is administered in combination with other compounds, the timing of administration, the tissue to which it is administered, and the physical delivery system with which it is carried.

As used herein, "treatment" or "treating" refers to a method for obtaining beneficial or desired results (including but not limited to therapeutic effects and/or prophylactic effects) with respect to a disease, disorder, or medical condition. Therapeutic benefit means eradicating or ameliorating the underlying disorder being treated. Furthermore, therapeutic benefit is achieved by eradicating or ameliorating one or more physiological symptoms associated with the underlying disorder, thereby observing improvement in the individual, although this individual may still be afflicted with the underlying disorder. Prophylactic effects include delaying or eliminating the appearance of a disease or condition, delaying or eliminating the onset of symptoms of a disease or condition, slowing, halting, or reversing the progression of a disease or condition, or any combination thereof. In certain embodiments, for prophylactic benefit, the composition is administered to an individual at risk of developing a particular disease, or to an individual reporting one or more physiological symptoms of a disease, even if a diagnosis of this disease may not have been made.

As used herein, the terms "co-administration," "combination administration," and grammatical equivalents thereof encompass the administration of two or more agents to an animal (including humans), so that both agents and/or their metabolites are present in the individual at the same time. Co-administration includes simultaneous administration in separate compositions, administration at different times in separate compositions, or administration in a composition in which both agents are present.

"Pharmaceutically acceptable salts" includes both acid addition salts and base addition salts.

"Pharmaceutically acceptable acid addition salts" refers to those salts that retain the biological effectiveness of the free base, being biologically tolerable, or being otherwise biologically suitable for administration to an individual. Preferred pharmaceutically acceptable acid addition salts are those salts that are pharmacologically effective and suitable for contact with patient tissues without undue toxicity, irritation, or allergic response. Pharmaceutically acceptable acid addition salts are formed from the following: inorganic acids such as, but not limited to, hydrochloric acid, hydrobromic acid, sulfuric acid, nitric acid, phosphoric acid, and the like; and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecyl sulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecenoic acid, and the like.

"Pharmaceutically acceptable base addition salts" refers to those salts that retain the biological effectiveness of the free acid, being biologically tolerable, or being otherwise biologically suitable for administration to an individual. Preferred pharmaceutically acceptable base addition salts are those salts that are pharmacologically effective and suitable for contact with patient tissues without undue toxicity, irritation, or allergic response. Pharmaceutically acceptable base addition salts are prepared by adding an inorganic or organic base to the free acid. Salts derived from inorganic bases include, but are not limited to, sodium salts, potassium salts, lithium salts, ammonium salts, calcium salts, magnesium salts, iron salts, zinc salts, copper salts, manganese salts, aluminum salts, and the like. Preferred inorganic salts are ammonium salts, sodium salts, potassium salts, calcium salts, and magnesium salts. Salts derived from organic bases include, but are not limited to, salts of primary, secondary, tertiary amines and substituted amines including naturally occurring substituted amines, cyclic amines, and basic ion exchange resins, for example, ammonia, isopropylamine, trimethylamine, diethylamine, triethylamine, tripropylamine, diethanolamine, ethanolamine, dimethylethanolamine, 2-dimethylaminoethanol, 2-ethylaminoethanol, dicyclohexylamine, lysine, arginine, histidine, caffeine, procaine, hydrabamine, choline, betaine, phenylethylbenzylamine, benzathine, ethylenediamine, glucosamine, meglumine, theobromine, triethanolamine, tromethamine, purines, piperazine, piperidine, N-ethylpiperidine, polyamine resins, and the like.

In some embodiments, pharmaceutically acceptable salts include quaternary ammonium salts, such as quaternary amine alkyl halide salts (e.g., methyl bromide).

The terms "antagonist" and "inhibitor" are used interchangeably, and they refer to a compound having the ability to inhibit the biological function of a target protein, whether by inhibiting the activity or expression of the protein (e.g., NLRP3 inflammasome or NEK7) or by inhibiting the association of the NLRP3 inflammasome with NEK7. Thus, the terms "antagonist" and "inhibitor" are defined in the context of the biological role of the target protein. Although preferred antagonists herein particularly interact with (e.g., bind to) the target, compounds that inhibit the biological activity of the target protein by interacting with other members of the signal transduction pathway in which the target protein is a member are also particularly included within this definition. Preferred biological activities inhibited by the antagonist are associated with tumor development, growth, or spread.

As used herein, the term "agonist" refers to a compound having the ability to initiate or enhance the biological function of a target protein, whether by inhibiting the activity or expression of the target protein or not. Thus, the term "agonist" is defined in the context of the biological role of the target polypeptide. Although preferred agonists herein particularly interact with (e.g., bind to) the target, compounds that initiate or enhance the biological activity of the target polypeptide by interacting with other members of the signal transduction pathway in which the target polypeptide is a member are also particularly included within this definition.

"Signal transduction" is the process by which stimulatory or inhibitory signals are transmitted to and within a cell to elicit an intracellular response.

The term "selective inhibition" or "selectively inhibit" refers to the ability of a biologically active agent to preferentially reduce the target signaling activity compared to off-target signaling activity, through direct or indirect interaction with the target.

"Individual" refers to an animal, for example, a mammal, e.g., a human. The methods described herein are useful for human therapy and veterinary applications. In some embodiments, the individual is a mammal, and in some embodiments, the individual is a human.

"Mammal" includes humans and livestock animals as well as non-livestock animals, wherein, livestock animals such as laboratory animals and household pets (e.g., cats, dogs, pigs, cows, sheep, goats, horses, rabbits), and non-livestock animals such as wildlife, and the like.

"Prodrug" is intended to denote a compound that can be converted under physiological conditions or by solvolysis to a biologically active compound described herein (e.g., a compound of structure (I)). Thus, the term "prodrug" refers to a pharmaceutically acceptable precursor of a biologically active compound. In some aspects, the prodrug is inert when administered to a subject, but is converted in vivo to the active compound, for example, by hydrolysis. Prodrug compounds often provide advantages of solubility, tissue compatibility, or delayed release in mammalian organisms. The term "prodrug" is also intended to include any covalently bonded carriers that release the active compound in vivo when such prodrug is administered to a mammalian subject. Prodrugs of active compounds as described herein are generally prepared by modifying functional groups present in the active compound in such a way that the modification is cleaved, either in routine manipulation or in vivo, to the parent active compound. Prodrugs include compounds in which a hydroxy, amino, or thiol group is bonded to any group that, when the prodrug of the active compound is administered to a mammalian subject, cleaves to form a free hydroxy, free amino, or free thiol group, respectively. Examples of prodrugs include, but are not limited to, acetate, formate, and benzoate derivatives of hydroxy functional groups in the active compound, or acetamide, formamide, and benzamide derivatives of amine functional groups, and the like.

The term "in vivo" refers to events that occur within an individual's body.

The embodiments disclosed herein are also intended to encompass all pharmaceutically acceptable compounds of structure (I).

Certain embodiments are also intended to include in vivo metabolic products of the disclosed compounds. Such products may result from, for example, oxidation, reduction, hydrolysis, amidation, esterification, and the like of the administered compound, primarily due to enzymatic processes. Accordingly, embodiments include compounds produced by a method comprising administering a compound of the present disclosure to a mammal for a period of time sufficient to produce a metabolic product thereof. Such products are typically identified by administering a detectable dose of a radiolabeled compound of the present disclosure to an animal (e.g., rat, mouse, guinea pig, monkey) or to a human, allowing sufficient time for metabolism to occur, and isolating its conversion products from urine, blood, or other biological samples.

"Stable compound" and "stable structure" are intended to mean a compound that is sufficiently stable to survive isolation to a useful degree of purity from a reaction mixture and formulation into an effective therapeutic agent.

Typically, crystallization produces a solvate of the compounds disclosed herein. As used herein, the term "solvate" refers to an aggregate comprising one or more molecules of a compound of the present disclosure with one or more molecules of a solvent. In some embodiments, the solvent is water, in which case the solvate is a hydrate. Alternatively, in other embodiments, the solvent is an organic solvent. Thus, the compounds of the present disclosure may exist as hydrates, including monohydrates, dihydrates, hemihydrates, sesquihydrates, trihydrates, tetrahydrates, and the like, as well as corresponding solvated forms. The compounds of the present disclosure may be true solvates, while in other cases, the compounds of the present disclosure may merely retain exogenous water or be water plus some exogenous solvent mixture.

"Optional" or "optionally" means that the event or circumstance subsequently described may or may not occur, and means that the description includes instances where the event or circumstance occurs and instances where it does not. For example, "optionally substituted aryl" means that the aryl group may or may not be substituted, and that the description includes both substituted aryl groups and aryl groups having no substituents.

"Pharmaceutical composition" refers to a formulation of a compound of the present disclosure with a medium generally accepted in the art for the delivery of the compound of the present disclosure to a mammal, e.g., a human. Such media include all pharmaceutically acceptable carriers, diluents, or excipients therefor.

"Pharmaceutically acceptable carrier, diluent, or excipient" includes, but is not limited to, any adjuvant, carrier, excipient, glidant, sweetening agent, diluent, preservative, dye/colorant, flavor enhancer, surfactant, wetting agent, dispersant, suspending agent, stabilizer, isotonic agent, solvent, or emulsifier.

"Stereoisomers" refers to compounds composed of the same atoms bonded by the same bonds but having different three-dimensional structures that are non-interchangeable. The present disclosure contemplates various stereoisomers and mixtures thereof and includes "enantiomers," which refers to two stereoisomers whose molecules are non-superimposable mirror images of one another.

The compounds of the present disclosure (i.e., compounds of structure (I)) or pharmaceutically acceptable salts thereof may contain one or more geometric asymmetric centers and thus may give rise to stereoisomers, such as enantiomers, diastereomers, and other stereoisomeric forms, which are defined in terms of absolute stereochemistry as (R)- or (S)-, or (D)- or (L)- for amino acids. Embodiments therefore include all such possible isomers, as well as their racemic and optically pure forms. Optically active (+) and (-), (R)- and (S)-, or (D)- and (L)-isomers may be prepared using chiral synthons or chiral reagents, or resolved using conventional techniques, for example, chromatography and fractional crystallization. Conventional techniques for the preparation/separation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other geometric asymmetric centers, unless otherwise specified, it is intended that the compounds include both E and Z geometric isomers. Likewise, all tautomeric forms are also intended to be included.

Embodiments of the present disclosure include all manners of rotamers and conformationally restricted states of the compounds of the invention. Atropisomers are also included, which are stereoisomers resulting from hindered rotation around a single bond, where the energy difference caused by steric strain or other contributing factors creates a high enough rotational barrier to allow the separation of individual conformational isomers. By way of example, certain compounds of the present disclosure may exist as a mixture of atropisomers, or may be purified or enriched to exist as a single atropisomer.

In some embodiments, the compound of structure (I) is a mixture of enantiomers or diastereomers. In other embodiments, the compound of structure (I) is substantially a single enantiomer or diastereomer.

"Tautomer" refers to proton transfer from one atom of a molecule to another atom of the same molecule. Embodiments therefore include tautomers of the disclosed compounds.

"NLRP3-mediated disorder" refers to a disease or pathological process associated with the activation and dysfunction of NLRP3, including autoimmune diseases, inflammatory disorders, cardiovascular diseases, neurodegenerative disorders, bacterial and viral infections, allergies, asthma, pancreatitis, multiple organ failure, kidney disease, platelet aggregation, cancer, transplantation, sperm motility, erythrocyte deficiency, transplant rejection, lung injury, respiratory diseases, and ischemic conditions; particularly, including Type II diabetes, atherosclerosis, Alzheimer's disease, aging, fatty liver, metabolic syndrome, asthma, psoriasis, obesity, acute and chronic tissue damage caused by infection, gout, arthritis, macular degeneration, enteritis, hepatitis, peritonitis, silicosis, UV-induced skin sunburn, contact hypersensitivity, sepsis, cancer, neurodegenerative diseases, multiple sclerosis, and Muckle-Wells syndrome, etc. The NLRP3-mediated disorder includes rheumatoid arthritis, psoriatic arthritis, osteoarthritis, systemic lupus erythematosus, lupus nephritis, ankylosing spondylitis, osteoporosis, systemic sclerosis, multiple sclerosis, psoriasis, Type I diabetes, Type II diabetes, inflammatory bowel disease (Crohn's disease and ulcerative colitis), hyperimmunoglobulinemia D and periodic fever syndrome, cryopyrin-associated periodic syndromes, Schnitzler syndrome, systemic juvenile idiopathic arthritis, adult-onset Still's disease, gout, pseudogout, SAPHO syndrome, Castleman's disease, sepsis, stroke, atherosclerosis, celiac disease, DIRA (deficiency of IL-1 receptor antagonist), Alzheimer's disease, Parkinson's disease, and cancer.

### Compound

The present application provides a compound of Formula I 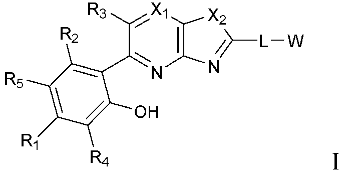
or a pharmaceutically acceptable salt, hydrate, solvate, active metabolite, polymorph, isotopically labeled compound, isomer, or prodrug thereof,
wherein, X₁ is selected from the group consisting of -N- and -CR₆-;
X₂ is selected from the group consisting of -O-, -S-, and -NR₇-;
R₁ is selected from the group consisting of C₂₋₈ alkynyl optionally substituted with one or two R₁₁, C₂₋₈ alkenyl optionally substituted with one or two R₁₁, C₂₋₈ haloalkynyl optionally substituted with one or two R₁₁, and C₂₋₈ haloalkenyl optionally substituted with one or two R₁₁; wherein each R₁₁ is independently selected from the group consisting of hydroxy and NR'R", and each of R' and R" is independently selected from the group consisting of hydrogen and C₁₋₆ alkyl;
R₂ is selected from the group consisting of hydrogen, halogen, cyano, C₁₋₈ alkyl, C₁₋₈ haloalkyl, C₁₋₈ alkoxy, and C₁₋₈ haloalkoxy; R₃ is selected from the group consisting of hydrogen, halogen, and C₁₋₈ alkyl; wherein at least one of R₂ and R₃ is not hydrogen;
each of R₄ and R₅ is independently selected from the group consisting of hydrogen, halogen, cyano, C₁₋₈ alkyl, C₁₋₈ haloalkyl, C₁₋₈ alkoxy, and C₁₋₈ haloalkoxy;
R₆ is selected from the group consisting of hydrogen, C₁₋₈ alkyl, C₁₋₈ haloalkyl, C₁₋₈ alkoxy, and C₁₋₈ haloalkoxy;
R₇ is selected from the group consisting of hydrogen, C₁₋₈ alkyl, C₁₋₈ haloalkyl, and C₁₋₈ alkoxy;
L is selected from the group consisting of -NR₈-, -O-, -S-, and -CR₉R₁₀-;
R₈ is selected from the group consisting of hydrogen, C₁₋₈ alkyl, C₁₋₈ haloalkyl, C₁₋₈ alkoxy, and C₁₋₈ haloalkoxy;
each of R₉ and R₁₀ is independently selected from the group consisting of hydrogen, hydroxy, C₁₋₈ alkyl, C₁₋₈ haloalkyl, C₁₋₈ alkoxy, and C₁₋₈ haloalkoxy; or R₉ and R₁₀, together with the C atom to which they are attached, form a C₃₋₈ cyclic hydrocarbyl group optionally substituted with R₀, wherein R₀ is selected from the group consisting of halogen, hydroxy, C₁₋₃ alkyl, and C₁₋₃ alkoxy;
W is a 4- to 10-membered heterocyclyl optionally substituted with one or two R^{W}, said 4-to 10-membered heterocyclyl contains up to 3 heteroatoms independently selected from the group consisting of N and O, and the maximum number of O heteroatom is 1, wherein each R^{W} is independently selected from the group consisting of hydrogen, oxo, hydroxy, hydroxy C₁₋₈ alkyl, C₁₋₈ alkyl, C₁₋₈ haloalkyl, C₁₋₈ alkoxy, C₁₋₈ haloalkoxy, C₃₋₈ cycloalkyl, C₃₋₈ halocycloalkyl, and NR^{w1}R^{w2}; wherein each of R^{w1} and R^{w2} is independently selected from the group consisting of hydrogen and C₁₋₆ alkyl.

In one embodiment, R₁ is C₂₋₈ alkynyl or C₂₋₈ alkenyl.

In one embodiment, R₁ is ethynyl, vinyl, or propynyl (including, for example, prop-1-yn-1-yl, prop-1-yn-3-yl).

In one embodiment, R₄ and R₅ are hydrogen.

In one embodiment, one of R₂ and R₃ is C₁₋₃ alkyl, for example methyl, and the other is hydrogen.

In one embodiment, X₁ is CH.

In one embodiment, X₂ is -O-, -S-, -NH-, or -N C₁₋₃ alkyl-.

In one embodiment, X₂ is -O-.

In one embodiment, L is -NH-, -O-, -S-, or - CH₂-.

In one embodiment, L is -NH-.

In one embodiment, W is a 4- to 8-membered heterocyclyl optionally substituted with one or two R^{W}, said 4- to 8-membered heterocyclyl contains up to 3 heteroatoms independently selected from the group consisting of N and O, and the maximum number of O heteroatom is 1, wherein R^{W} is each independently selected from the group consisting of hydrogen, oxo, hydroxy, hydroxy C₁₋₆ alkyl, C₁₋₆ alkyl, C₁₋₆ haloalkyl, C₁₋₆ alkoxy, C₁₋₆ haloalkoxy, C₃₋₆ cyclic hydrocarbyl group, C₃₋₆ halo cyclic hydrocarbyl group, and NR^{w1}R^{w2}; wherein each of R^{w1} and R^{w2} is independently selected from the group consisting of hydrogen and C₁₋₆ alkyl. The 4- to 8-membered heterocyclyl may be a monocyclic heterocyclyl, or a fused heterocyclyl, for example, oxetanyl, azetidinyl, tetrahydrofuranyl, pyrrolidinyl, tetrahydropyranyl, piperidinyl, morpholinyl, piperazinyl, dioxanyl, and the like.

In one embodiment, W is a 6-membered heterocyclyl containing a single N heteroatom, e.g., piperidinyl, optionally substituted with one or two R^{W}, wherein each R^{W} is independently selected from the group consisting of C₁₋₆ alkyl, hydroxy C₁₋₆ alkyl, and C₃₋₆ cycloalkyl.

In one embodiment, W is one of the following structural formulae: 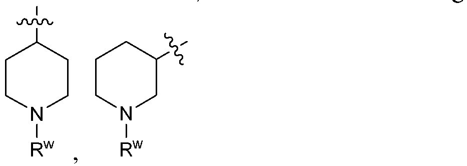 wherein, each R^{W} is independently selected from the group consisting of C₁₋₃ alkyl, hydroxy C₁₋₃ alkyl, and C₃₋₆ cycloalkyl.

In particular, W is one of the following structural formulae:

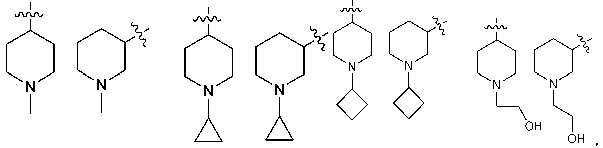

In one embodiment, the compound is selected from one of the following compounds:

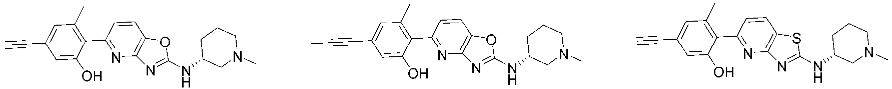

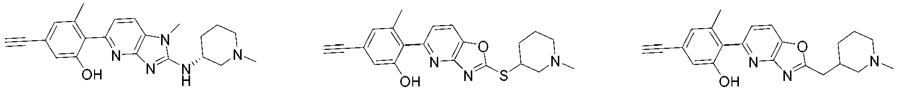

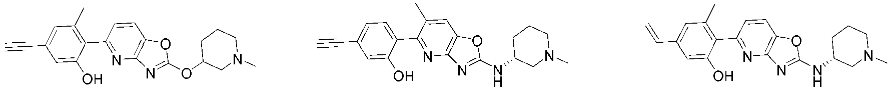

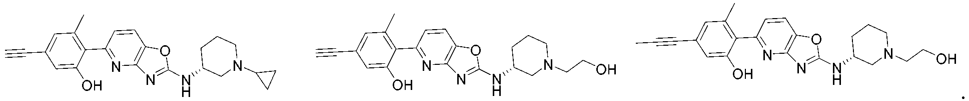

### Pharmaceutical Compositions

The present disclosure also relates to a pharmaceutical composition. The pharmaceutical composition comprises any one (or more) of the foregoing compounds and a pharmaceutically acceptable carrier. In some embodiments, the pharmaceutical composition is formulated for oral administration. In other embodiments, the pharmaceutical composition is formulated for injection. In further embodiments, the pharmaceutical composition comprises a compound disclosed herein and an additional therapeutic agent (e.g., an anticancer agent). Non-limiting examples of such therapeutic agents are described below.

Suitable ways for administration include, but are not limited to, oral, intravenous, rectal, aerosol, parenteral, ophthalmic, pulmonary, transmucosal, transdermal, vaginal, otic, nasal, and topical administration. Additionally, by way of example only, parenteral delivery includes intramuscular, subcutaneous, intravenous, intramedullary injection, as well as intrathecal, direct intraventricular, intraperitoneal, intralymphatic, and intranasal injections.

In certain embodiments, the compounds described herein are administered topically rather than systemically, for example, by injecting the compound directly into an organ, typically in the form of a depot formulation or sustained release formulation. In specific embodiments, long-acting formulations are administered by implantation (e.g., subcutaneously or intramuscularly) or by intramuscular injection. Furthermore, in other embodiments, the compounds are delivered in a targeted drug delivery system, for example, in liposomes coated with organ-specific antibodies. In such embodiments, the liposomes target the organ and are selectively taken up by the organ. In other embodiments, the compounds as described herein are provided in the form of a rapid release formulation, an extended release formulation, or an intermediate release formulation. In other embodiments, the compounds described herein are administered topically.

In the treatment methods according to embodiments of the present invention, an effective amount of at least one compound of structure (I) is administered to an individual suffering from or diagnosed with such a disease, disorder, or medical condition. The effective amount or dose may be determined by methods such as modeling, dose escalation studies, or clinical trials, taking into account, for example, the mode or route of administration or drug delivery, the pharmacokinetics of the agent, the severity and course of the disease, disorder, or condition, the individual's prior or ongoing therapy, the individual's health status and response to drugs, and the judgment of the treating physician.

The compound according to the present disclosure are effective over a wide dosage range. For example, in the treatment of adults, dosages of 10 to 5000 mg/day, 100 to 5000 mg/day, 1000 to 4000 mg/day, and 1000 to 3000 mg/day are examples of dosages used in some embodiments. The exact dosage will depend on the route of administration, the form of the compound administered, the subject to be treated, the body weight of the subject to be treated, and the preference and experience of the attending physician.

In some embodiments, the compound of the present disclosure is administered as a single dose. Typically, this administration will be by injection, for example, intravenous injection, for rapid introduction of the agent. However, other routes are used as appropriate. A single dose of a compound of the present disclosure may also be used for the treatment of acute conditions.

In some embodiments, the compound described herein is formulated as a pharmaceutical composition. In specific embodiments, the pharmaceutical composition is formulated in a conventional manner using one or more physiologically acceptable carriers, which comprise excipients and auxiliaries that facilitate the processing of the disclosed compounds into pharmaceutically usable preparations. The appropriate formulation depends on the chosen route of administration.

Provided herein is a pharmaceutical composition comprising one or more compounds of structure (I) and a pharmaceutically acceptable carrier.

Provided herein a pharmaceutical composition comprising one or more compounds selected from the group consisting of compounds of structure (I) and a pharmaceutically acceptable diluent, excipient, and carrier. In certain embodiments, the compound is administered as a pharmaceutical composition, wherein one or more compounds selected from the group consisting of compounds of structure (I) are admixed with other active ingredients, as in combination therapy. All combinations of active substances set forth in the combination therapy section below and all combinations of active substances set forth throughout this disclosure are encompassed herein. In specific embodiments, the pharmaceutical composition comprises one or more compounds of structure (I).

A pharmaceutical composition as used herein refers to a mixture of one or more compounds selected from the group consisting of compounds of structure (I) with other chemical components, such as carriers, stabilizers, diluents, dispersing agents, suspending agents, thickening agents, and/or excipients. In certain embodiments, the pharmaceutical composition facilitates administration of the compound to an organism. In some embodiments, a therapeutically effective amount of one or more compounds selected from the group consisting of compounds of structure (I) provided herein is administered in a pharmaceutical composition to a mammal suffering from a disease, disorder, or medical condition to be treated. In specific embodiments, the mammal is a human. In certain embodiments, the therapeutically effective amount varies depending on the severity of the disease, the age and relative health conditions of the individual, the efficacy of the compound used, and other factors. The compounds described herein are used alone or in combination with one or more therapeutic agents as a component of a mixture.

In another embodiment, the compounds described herein are formulated for oral administration. The compounds described herein are formulated by combining the active compound with, for example, a pharmaceutically acceptable carrier or an excipient. In various embodiments, the compounds described herein are formulated into oral dosage forms, including (by way of example only) tablets, powders, pills, dragees, capsules, liquids, gels, syrups, elixirs, slurries, suspensions, and the like.

In certain embodiments, pharmaceutical preparations for oral administration are obtained by the follows: mixing one or more solid excipients with one or more compounds described herein, optionally grinding the resulting mixture, and, if necessary, after adding suitable auxiliaries, processing the mixture of granules to obtain tablets or dragee cores. Suitable excipients are, in particular, fillers such as sugars, including lactose, sucrose, mannitol, or sorbitol; cellulose preparations, for example: corn starch, wheat starch, rice starch, potato starch, gelatin, tragacanth, methylcellulose, microcrystalline cellulose, hydroxypropyl methylcellulose, sodium carboxymethylcellulose; or other substances, for example: polyvinylpyrrolidone (PVP or povidone) or calcium phosphate. In specific embodiments, disintegrants are optionally added. By way of example only, disintegrants include cross-linked croscarmellose sodium, polyvinylpyrrolidone, agar, or alginic acid or a salt thereof such as sodium alginate.

### Use

The present disclosure also relates to methods of treating NLRP3-mediated disorders, comprising administering to a subject in need thereof a therapeutically effective amount of a compound of Formula I or a pharmaceutical composition of the present disclosure.

As evidenced by the disclosure, the compounds of the present disclosure possess NEK7 enzymatic inhibitory activity, and thus can be used for the treatment or prevention of NLRP3-mediated disorders, as well as for the manufacture of a pharmaceutical for the treatment or prevention of NLRP3-mediated disorders.

The host or patient may belong to any mammalian species, for example, primate species, particularly humans; rodents, including mice, rats, and hamsters; rabbits; horses, cows, dogs, cats, etc. Animal models are of interest for experimental research, providing models for the treatment of human diseases.

The NLRP3-mediated disorder may be selected from the group consisting of autoimmune diseases, inflammatory disorders, cardiovascular diseases, neurodegenerative disorders, bacterial and viral infections, allergies, asthma, pancreatitis, multiple organ failure, kidney disease, platelet aggregation, cancer, transplantation, sperm motility, erythrocyte deficiency, transplant rejection, lung injury, respiratory diseases, and ischemic conditions.

The NLRP3-mediated disorder may be selected from the group consisting of Type II diabetes, atherosclerosis, Alzheimer's disease, aging, fatty liver, metabolic syndrome, asthma, psoriasis, obesity, acute and chronic tissue damage caused by infection, gout, arthritis, macular degeneration, enteritis, hepatitis, peritonitis, silicosis, UV-induced skin sunburn, contact hypersensitivity, sepsis, cancer, neurodegenerative diseases, multiple sclerosis, and Muckle-Wells syndrome.

The NLRP3-mediated disorder may be selected from the group consisting of rheumatoid arthritis, psoriatic arthritis, osteoarthritis, systemic lupus erythematosus, lupus nephritis, ankylosing spondylitis, osteoporosis, systemic sclerosis, multiple sclerosis, psoriasis, Type I diabetes, Type II diabetes, inflammatory bowel disease (Crohn's disease and ulcerative colitis), hyperimmunoglobulinemia D and periodic fever syndrome, cryopyrin-associated periodic syndromes, Schnitzler syndrome, systemic juvenile idiopathic arthritis, adult-onset Still's disease, gout, pseudogout, SAPHO syndrome, Castleman's disease, sepsis, stroke, atherosclerosis, celiac disease, DIRA (deficiency of IL-1 receptor antagonist), Alzheimer's disease, Parkinson's disease, and cancer.

The following examples and formulations further illustrate and exemplify the compounds of the present disclosure, as well as methods of preparing and testing such compounds. It should be understood that the scope of the present disclosure is not limited in any way by the scope of the following examples and formulations. In the following examples, and throughout the specification and claims, unless otherwise indicated, molecules with a single stereocenter exist as racemic mixtures. Unless otherwise indicated, those molecules with two or more stereocenters exist as racemic mixtures of diastereoisomers. Single enantiomers/diastereomers may be obtained by methods known to those skilled in the art.

A part of compounds of the general formula I of the present invention can be prepared by those skilled in the art of organic synthesis using standard methods in the art according to the following scheme:

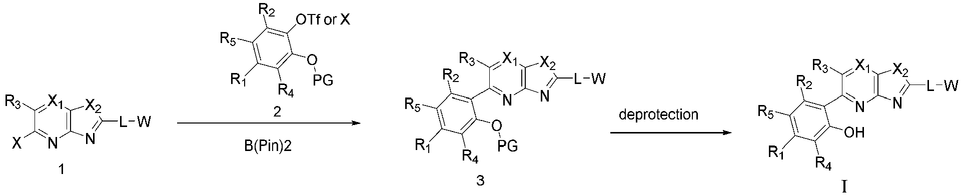

Compound of formula 1 and compound of formula 2 undergo a coupling reaction in the presence of bis(pinacolato)diboron under palladium-catalyzed conditions to afford compound of formula 3, which is then deprotected to yield the compound of formula I of the present application.

The invention is illustrated in more detail by specific examples. The following examples are provided for illustrative purposes and should not be construed as limiting the invention in any way. Those skilled in the art will readily recognize that a variety of non-critical parameters can be changed or modified to yield substantially the same results. The compounds of the following examples were found to be NLRP3 inhibitors according to one or more of the assays described herein.

### Example 1:

### (R)-5-Ethynyl-3-methyl-2-(2-((1-methylpiperidin-3-yl)amino)oxazolo[4,5-b]pyridin-5-yl)phenol

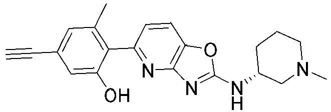

### Synthetic route:

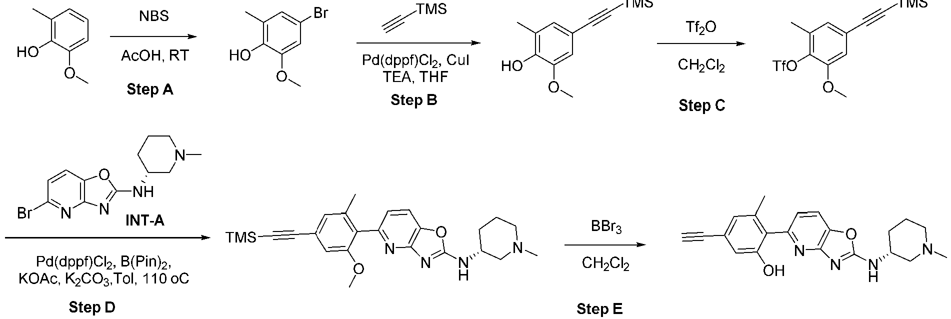

### Step A: 4-Bromo-2-methoxy-6-methylphenol

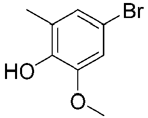

To a 250 mL round-bottomed flask were added 10.0 g (72.5 mmol, 1.0 eq) of 2-methoxy-6-methylphenol, 12.9 g (72.5 mmol, 1.0 eq) of NBS, and 50 mL of acetic acid. The reaction was stirred at room temperature for 4 hours. LCMS indicated complete conversion of the starting material. The reaction mixture was concentrated under reduced pressure to give a crude product. The crude product was purified by silica gel column chromatography (petroleum ether:ethyl acetate = 8:1) to give 4-bromo-2-methoxy-6-methylphenol (12.0 g, yield = 77%) as a yellow oily product.

LC-MS: (M-H)⁻; m/z=216.9.

### Step B: 2-Methoxy-6-methyl-4-((trimethylsilyl)ethynyl)phenol

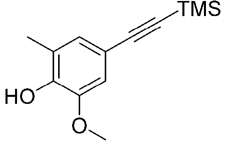

To a 50 mL three-necked flask were added 6.00 g (27.6 mmol, 1.0 eq) of 4-bromo-2-methoxy-6-methylphenol, 27.2 g (276 mmol, 10.0 eq) of trimethylsilylacetylene, 2.02 g (2.76 mmol, 0.1 eq) of Pd(dppf)Cl₂, 1.05 g (5.52 mmol, 0.2 eq) of CuI, 19.2 mL (138 mmol, 5.0 eq) of triethylamine, and 40 mL of tetrahydrofuran. The reaction solution was stirred at 80 °C for 12 hours under nitrogen atmosphere. After LCMS indicated completion of the reaction, the reaction solution was filtered hot through Celite, and the Celite pad was washed with ethyl acetate (50 mL). The resulting filtrate was extracted with water (25 mL), and the organic phase was separated. The aqueous phase was further extracted with ethyl acetate (25 mL). The organic phases were combined and concentrated under reduced pressure to give a brown oily crude product. The crude product was purified by reverse phase flash chromatography (5%~80% ACN in H₂O) to give 2-methoxy-6-methyl-4-((trimethylsilyl)ethynyl)phenol (1.20 g, yield = 19%) as a yellow solid.

LC-MS: (M-H)⁻; m/z=233.2.

### Step C: 2-Methoxy-6-methyl-4-((trimethylsilyl)ethynyl)phenyl trifluoromethanesulfonate

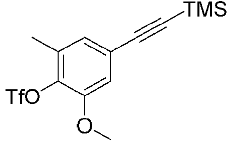

To a 50 mL round-bottomed flask were added 1.20 g (5.12 mmol, 1.0 eq) of 2-methoxy-6-methyl-4-((trimethylsilyl)ethynyl)phenol, 0.98 g (7.68 mmol, 1.5 eq) of pyridine, and 15 mL of dichloromethane as a solvent. Then, 1.73 g (6.14 mmol, 1.2 eq) of trifluoromethanesulfonic anhydride was slowly added dropwise at 0 °C. After the addition, the reaction was allowed to warm to room temperature and stirred for 2 hours, monitored by LCMS. After completion, 2 mL of water was added to the reaction mixture to quench the reaction. The organic phase was separated, washed with saturated brine (2 mL), dried over anhydrous sodium sulfate, and concentrated under reduced pressure to give 2-methoxy-6-methyl-4-((trimethylsilyl)ethynyl)phenyl trifluoromethanesulfonate (1.30 g, yield = 69%) as a yellow solid.

LC-MS: (M-H)⁻; m/z=365.1.

### Step D:

### (R)-5-(2-Methoxy-6-methyl-4-((trimethylsilyl)ethynyl)phenyl)-N-(1-methylpiperidin-3-yl)oxazolo[4,5-b]pyridin-2-amine

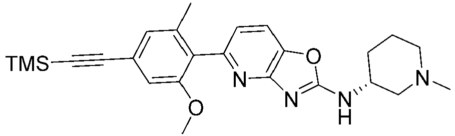

To a 25 mL three-necked flask were added 476 mg (1.53 mmol, 1.4 eq) of (R)-5-bromo-N-(1-methylpiperidin-3-yl)oxazolo[4,5-b]pyridin-2-amine, 79.9 mg (0.109 mmol, 0.1 eq) of Pd(dppf)Cl₂, 1109 mg (4.37 mmol, 4.0 eq) of bis(pinacolato)diboron, 536 mg (5.46 mmol, 5.0 eq) of potassium acetate, and 10 mL of toluene. The mixture was stirred at 110 °C for 1 hour under nitrogen atmosphere. LCMS indicated formation of the intermediate. To the reaction mixture were added 400 mg (1.09 mmol, 1.0 eq) of 2-methoxy-6-methyl-4-((trimethylsilyl)ethynyl)phenyl trifluoromethanesulfonate, 754 mg (5.46 mmol, 5.0 eq) of K₂CO₃, and 1 mL of water. The reaction was continued under nitrogen atmosphere for 2 hours. After LCMS indicated complete conversion of the starting material, ethyl acetate (50 mL) and water (20 mL) were added to the reaction mixture. The organic phase was separated, and the aqueous phase was further extracted with ethyl acetate (50 mL). The organic phases were combined and concentrated under reduced pressure. Reverse phase flash chromatography purification (5%~80% ACN in H₂O) gave 5-(2-methoxy-6-methyl-4-((trimethylsilyl)ethynyl)phenyl)-N-(1-methylpiperidin-3-yl)oxazolo[4,5-b]pyridin-2-amine (125 mg, 0.279 mmol, 26%, 92% purity) as a yellow solid crude product. A 15 mg of the crude product was purified by preparative chromatography (mobile phase A: 10 M ammonium bicarbonate; mobile phase B: acetonitrile; gradient: 15%-50% B, 20 min; flow rate: 19 mL/min) and lyophilized to give a white solid (7.00 mg, yield = 13%).

LC-MS: (M+H)⁺; m/z = 449.4;

¹H-NMR (400 MHz, DMSO-*d₆*) δ 8.43 (d, *J =* 7.9 Hz, 1H), 7.70 (d, *J* = 8.0 Hz, 1H), 7.02 (s, 1H), 6.96 (d, *J =* 1.4 Hz, 1H), 6.82 (d, *J* = 8.0 Hz, 1H), 3.76 (s, 1H), 3.65 (s, 3H), 2.91 (d, *J* = 10.3 Hz, 1H), 2.60 (d, *J* = 10.8 Hz, 1H), 2.19 (s, 3H), 1.96 (s, 3H), 1.91 (d, *J* = 11.3 Hz, 2H), 1.77 - 1.65 (m, 1H), 1.53 (q, *J =* 12.4, 12.0 Hz, 1H), 1.39 - 1.21 (m, 2H), 0.25 (s, 9H).

### Step E:

### (R)-5-Ethynyl-3-methyl-2-(2-((1-methylpiperidin-3-yl)amino)oxazolo[4,5-b]pyridin-5-yl)phenol

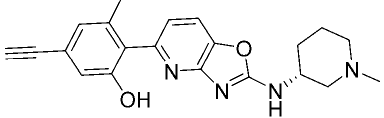

55.1 mg (123 µmol, 1.0 eq) of *(R)-*5-(2-methoxy-6-methyl-4-((trimethylsilyl)ethynyl)phenyl)-N-(1-methylpiperidin-3-yl)oxazolo[4,5-b]pyridin-2-amine was dissolved in 3 mL of anhydrous dichloromethane. The solution was cooled to -70 °C, and then 308 mg (1.23 mmol, 10.0 eq) of boron tribromide was added to the reaction mixture. After addition, the mixture was allowed to warm to room temperature naturally and stirred for 3 hours. LC-MS results confirmed complete conversion of the starting material. The mixture was cooled to 0 °C, and 1 mL of ammonia water (25%) was added to quench the reaction. The mixture was concentrated under reduced pressure to give a crude product. The crude product was purified by preparative chromatography (mobile phase A: 10 M ammonium bicarbonate; mobile phase B: acetonitrile; gradient: 15%-50% B, 20 min; flow rate: 19 mL/min) and lyophilized to give a white solid (5.40 mg, yield = 12%).

LC-MS: (M+H)⁺; m/z=363.2;

¹H-NMR (400 MHz, CDCl₃) *δ* 10.64 (br s, 1H), 7.56 (d, *J =* 8.2 Hz, 1H), 7.13 (d, *J =* 8.3 Hz, 1H), 7.02 (s, 1H), 6.97 (s, 1H), 4.22 (s, 1H), 3.05 (s, 1H), 2.84 - 2.63 (m, 2H), 2.56 - 2.47 (m, 1H), 2.43 (s, 3H), 2.33 (s, 3H), 2.25 - 2.16 (m, 1H), 2.12 - 1.68 (m, 5H).

### Intermediate (INT-A):

### (R)-5-Bromo-N-(1-methylpiperidin-3-yl)oxazolo[4,5-b]pyridin-2-amine

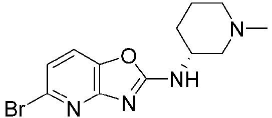

### Synthetic route:

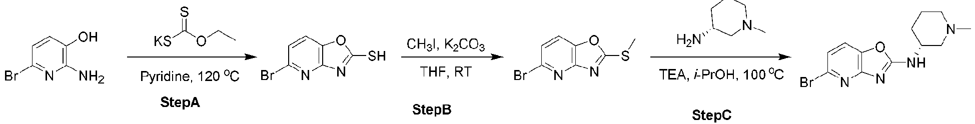

### Step A: 5-Bromooxazolo[4,5-b]pyridine-2-thiol

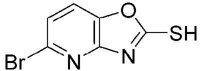

5.00 g (26.4 mmol, 1.0 eq) of 2-amino-6-bromopyridin-3-ol, 4.66 g (29.0 mmol, 1.1 eq) of potassium ethylxanthate, and 50 mL of pyridine were placed in a flask. The mixture was purged with nitrogen three times and stirred at 100 °C overnight. LCMS showed complete reaction of the starting material. The reaction solution was added with water (50 mL), adjusted to pH 4-5 with 10% aqueous hydrochloric acid, and extracted with ethyl acetate (50 mL) three times. The combined organic layers were washed with saturated brine (20 mL) three times, dried over anhydrous sodium sulfate, filtered by suction, and concentrated to give a yellow solid crude product (4.5 g, yield = 73%).

LC-MS: (M-H)⁺; m/z=230.8.

### Step B: 5-Bromo-2-(methylthio)oxazolo[4,5-b]pyridine

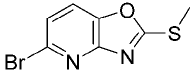

4.50 g (19.4 mmol, 1.0 eq) of 5-bromooxazolo[4,5-b]pyridine-2-thiol, 5.92 g (42.8 mmol, 2.2 eq) of potassium carbonate, and 50 mL of tetrahydrofuran were added to a 100 mL reaction flask. The mixture was cooled to 0 °C, and 4.15 g (29.2 mmol, 1.5 eq) of iodomethane was slowly added dropwise. The mixture was allowed to warm to room temperature naturally and stirred for 2 hours at room temperature. LCMS showed complete reaction of the starting material. The reaction solution was added with water (50 mL) and extracted with ethyl acetate (50 mL) three times. The combined organic layers were washed with saturated brine (20 mL) three times, dried over anhydrous sodium sulfate, filtered by suction, and concentrated. The crude product was purified by column chromatography (petroleum ether:ethyl acetate = 10:1) to give the product as a white solid (4.50 g, yield = 94%).

LC-MS: (M+H)⁺; m/z=244.8.

### Step C: (R)-5-Bromo-N-(1-methylpiperidin-3-yl)oxazolo[4,5-b]pyridin-2-amine

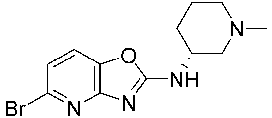

800 mg (3.26 mmol, 1.0 eq) of 5-bromo-2-(methylthio)oxazolo[4,5-b]pyridine, 559 mg (4.86 mmol, 1.5 eq) of *(R)*-1-methylpiperidin-3-amine, 990 mg (9.79 mmol, 3.0 eq) of triethylamine, and 10 mL of isopropanol were added to a 50 mL sealed tube. The mixture was stirred at 100 °C overnight. LCMS showed complete reaction of the starting material. The reaction solution was concentrated to dryness, triturated with ethyl acetate, filtered, and dried to give the pure product as a yellow solid (800 mg, yield = 78%).

LC-MS: (M+H)⁺; m/z=311.1.

The following examples were prepared according to the experimental route and methods described in Example 1:

| | |
|---|---|
| Example 2: *(R)*-3-Methyl-2-(2-((1-methylpiperidin-3-yl)amino)oxazolo[4,5-b]pyridin-5-yl)-5-(prop-1-yn-1-yl)phenol | |
| 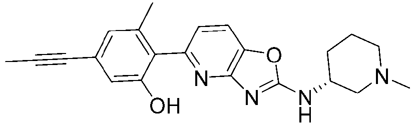 | LC-MS(ESI), m/z: [M+H] ⁺ =377.2. |
| Example 3: *(R)*-5-Ethynyl-3-methyl-2-(2-((1-methylpiperidin-3-yl)amino)thiazolo[4,5-b]pyridin-5-yl)phenol | |
| 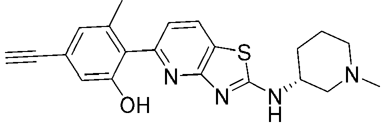 | LC-MS(ESI), m/z: [M+H] ⁺ =379.2. |
| Example 4: *(R)*-5-Ethynyl-3-methyl-2-(1-methyl-2-((1-methylpiperidin-3-yl)amino)-1H-imidazo[4,5-b]pyridin-5-yl)phenol | |
| 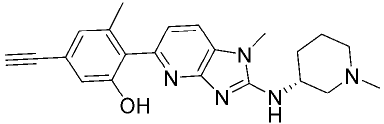 | LC-MS (ESI), m/z: [M+H]⁺ = 376.2. |
| Example 5: 5-Ethynyl-3-methyl-2-(2-((1-methylpiperidin-3-yl)thio)oxazolo[4,5-b]pyridin-5-yl)phenol | |
| 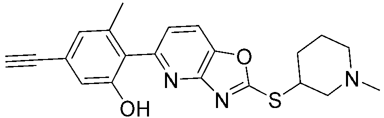 | LC-MS(ESI), m/z: [M+H] ⁺ =380.2. |
| Example 6: 5-Ethynyl-3-methyl-2-(2-((1-methylpiperidin-3-yl)methyl)oxazolo[4,5-b]pyridin-5-yl)phenol | |
| 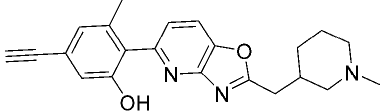 | LC-MS (ESI), m/z: [M+H]⁺ =330. |
| Example 7: 5-Ethynyl-3-methyl-2-(2-((1-methylpiperidin-3-yl)oxy)oxazolo[4,5-b]pyridin-5-yl)phenol | |
| 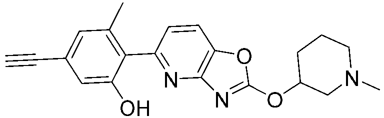 | LC-MS(ESI), m/z: [M+H] ⁺ =364.2. |
| Example 8: *(R)*-5-Ethynyl-2-(6-methyl-2-((1-methylpiperidin-3-yl)amino)oxazolo[4,5-b]pyridin-5-yl)phenol | |
| 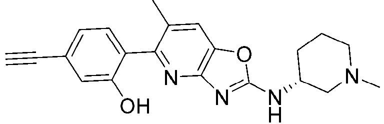 | LC-MS (ESI), m/z: [M+H]⁺ = 363.2. |
| Example 9: *(R)*-3-Methyl-2-(2-((1-methylpiperidin-3-yl)amino)oxazolo[4,5-b]pyridin-5-yl)-5-vinylphenol | |
| 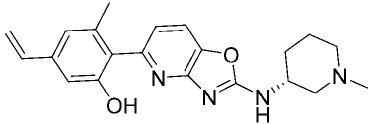 | LC-MS (ESI), m/z: [M+H]⁺ = 365.2. |
| Example 10: *(R)*-2-(2-((1-Cyclopropylpiperidin-3-yl)amino)oxazolo[4,5-b]pyridin-5-yl)-5-ethynyl-3-methylphenol | |
| 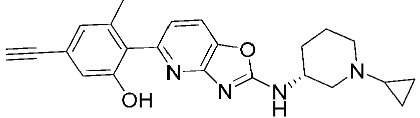 | LC-MS(ESI), m/z: [M+H] ⁺ =389.2. |
| Example 11: *(R)*-5-Ethynyl-2-(2-((1-(2-hydroxyethyl)piperidin-3-yl)amino)oxazolo[4,5-b]pyridin-5-yl)-3-methylphenol | |
| 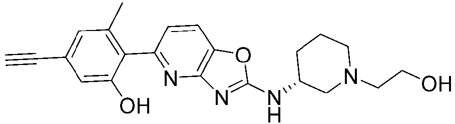 | ¹H-NMR (400 MHz, DMSO-*d*₆) δ 9.56 (s, 1H), 8.39 (d, *J =* 8.0 Hz, 1H), 7.70 (d*, J =* 8.1 Hz, 1H), 6.90 - 6.84 (m, 3H), 4.10 (s, 1H), 3.85 - 3.77 (m, 1H), 3.50 (t, *J* = 6.0 Hz, 2H), 2.99 - 2.96 (m, 1H), 2.68 - 2.64 (m, 1H), 2.44 (t, *J* = 6.1 Hz, 2H), 2.15 (d, *J =* 11.2 Hz, 2H), 2.01 (s, 3H), 1.91 - 1.87 (m, 1H), 1.75 - 1.69 (m, 1H), 1.57 - 1.48 (m, 1H), 1.45 - 1.37 (m, 1H). LC-MS(ESI), m/z: [M+H] ⁺ =393.2. |
| Example 12: *(R)*-2-(2-((1-(2-Hydroxyethyl)piperidin-3-yl)amino)oxazolo[4,5-b]pyridin-5-yl)-3-methyl-5-(prop-1-yn-1-yl)phenol | |
| 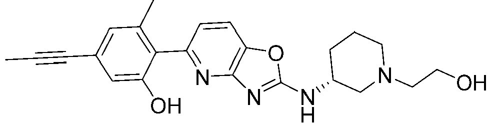 | LC-MS (ESI), m/z: [M+H]⁺ =407.2. |

### Biological Activity Experiment: IL-1β Secretion Test of THP-1 Cells

Experimental procedure: THP-1 cells were cultured in 1640 medium containing 10% heat-inactivated fetal bovine serum at a temperature of 37°C and a carbon dioxide concentration of 5%. At the start of the experiment, the cell suspension was gently shaken evenly and transferred to a centrifuge tube for counting. The required volume was taken and added to fresh subculture medium. In a 96-well plate, 40 µL of sterile water-diluted poly-L-lysine was added and incubated at 37°C & 5% CO₂ for 30 minutes, then washed twice with 100 µL. 50 ng/mL PMA (phorbol 12-myristate 13-acetate) were added to the THP-1 cell suspension, followed by 50,000 cells per well per 100 µL were seeded into the 96-well plate from Step 1 and incubated together at 37°C & 5% CO₂ for 24 hours. The medium in the 96-well plate was removed, and the cells were washed once with pre-warmed PBS (37°C). 85 µL of serum-free medium containing 25 ng/mL LPS was added, and the cells were incubated at 37°C & 5% CO₂ for 3 hours. 5 µL of compound at various concentrations (DMSO concentration uniformly 1‰) was added, and the cells were incubated at 37°C & 5% CO₂ for an additional 30 minutes. 5 µL of diluted Nigericin was added to achieve a working concentration of 5 µg/mL, and the cells were incubated at 37°C & 5% CO₂ for 1 hour. The cell supernatant was collected, stored at -80°C, and then the secretion of IL-1β was detected using an ELISA kit. The IL-1β concentration was calculated based on the standard curve, and the inhibition rate was calculated. The compound dose-response curve was fitted, and the IC₅₀ was calculated.

The specific results of the test are shown in Table 1 below:

**Table 1: IL-1β secretion Test of compounds on THP-1 cells**

| Example | IL-1β Release IC₅₀ (nM) |
|---|---|
| 1 | <5 |
| 2 | <5 |
| 3 | <10 |
| 4 | <20 |
| 5 | <20 |
| 6 | <10 |
| 7 | <10 |
| 8 | <20 |
| 9 | <5 |
| 10 | <5 |
| 11 | <5 |
| 12 | <5 |
| MCC950 | 46.97 |

MCC950 is a known potent and selective NLRP3 inhibitor, with the following specific structure:

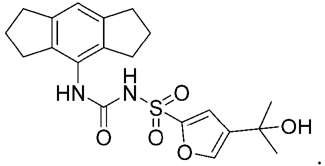

As shown by the data in the table, the compounds of the examples of the present invention exhibited significantly superior inhibitory activity on the induced release of IL-1β in THP-1 cells compared to the known NLRP3 inhibitor MCC950. The present patent invention provides a novel heterocyclic compound, which is an NLRP3 inhibitor and can be used for treating NLRP3-mediated diseases.

### Pharmacokinetic Test

Male Balb/c mice were divided into groups, with 3 mice in each group. The compound of Example 1 was dissolved in vehicle: 5% DMSO/10% Solutol/85% (20% Captisol in water). The compound of Example 1 was administered intravenously (2 mg/kg) and orally as a single gavage (10 mg/kg), respectively. Animals were fasted overnight before the experiment, with food withheld from 10 hours before administration until 4 hours after administration. Blood samples were collected at 0.0833, 0.25, 0.5, 1, 2, 4, 6, 8, and 24 hours after intravenous administration, and at 0.25, 0.5, 1, 2, 4, 6, 8, and 24 hours after oral administration. 0.3 mL of whole blood was collected under isoflurane anesthesia into heparinized tubes. Samples were centrifuged at 4000 rpm for 5 min at 4°C, and plasma was transferred to centrifuge tubes and stored at -80°C until analysis. Plasma samples were extracted using protein precipitation, and the extracts were analyzed by LC/MS/MS. The pharmacokinetic results are shown in Tables 2 and 3 below:

**Table 2: Pharmacokinetic parameters of the compound in mouse plasma after intravenous administration**

| **PK Parameter** | **Example 1** |
|---|---|
| C₀ (ng/mL) | 1727 |
| T_{1/2} (hr) | 8.85 |
| AUC₀₋ₜ (ng·hr/mL) | 6954 |
| Cl (mL/min/kg) | 4.20 |

**Table 3: Pharmacokinetic parameters of the compound in mouse plasma after oral administration**

| **PK Parameter** | **Example 1** |
|---|---|
| T_{1/2} (hr) | 6.71 |
| Tₘₐₓ (hr) | 1.33 |
| Cₘₐₓ (ng/mL) | 2947 |
| AUC₀₋ₜ (ng·hr/mL) | 31402 |
| Bioavailability (%) | 84.5% |

The data in the tables show that the compound of Example 1 of the present invention is well absorbed and bioavailable after oral gavage administration, with a bioavailability of 84.5%.

### Blood-Brain Distribution Experiment

Male Balb/c mice were divided into groups, with 3 mice in each group, and received a single intravenous administration of the compound of the example (2 mg/kg), respectively. Animals were fasted overnight before the experiment, with food withheld from 10 hours before administration until 4 hours after administration. Each mouse was sacrificed 0.5 hours after administration, and blood and brain tissue were collected. Samples were centrifuged at 4000 rpm for 5 min at 4°C, and plasma was transferred to centrifuge tubes and stored at -80°C until analysis. Plasma samples were extracted using protein precipitation, and the extracts were analyzed by LC/MS/MS. The results of the blood-brain distribution experiment are shown in Table 4. The data indicate that the compound of Example 1 of the present invention can cross the blood-brain barrier, whereas the compound of Example 11 does not readily cross the blood-brain barrier.

**Table 4: Concentration ratios of drugs in Blood-brain 0.5 h after administration in mice IV**

| **PK Parameter** | **Example 1** | **Example 11** |
|---|---|---|
| Drug in plasma (ng/mL) | 473 | 275 |
| Drug in brain tissue (ng/mL) | 348 | 54 |
| Concentration ratios of drugs in Brain/Blood | 73.6% | 19.7% |

The present application has been described above in conjunction with preferred embodiments; however, these embodiments are merely exemplary and serve only for illustrative purposes. On this basis, various substitutions and improvements may be made to the present application, all of which fall within the scope of protection of the present application.

## Claims

1. A compound of Formula I 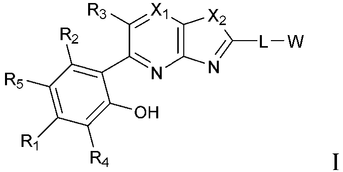
or a pharmaceutically acceptable salt, hydrate, solvate, active metabolite, polymorph, isotopically labeled compound, isomer, or prodrug thereof,
wherein, X₁ is selected from the group consisting of -N- and -CR₆-;
X₂ is selected from the group consisting of -O-, -S-, and -NR₇-;
R₁ is selected from the group consisting of C₂₋₈ alkynyl optionally substituted with one or two R₁₁, C₂₋₈ alkenyl optionally substituted with one or two R₁₁, C₂₋₈ haloalkynyl optionally substituted with one or two R₁₁, and C₂₋₈ haloalkenyl optionally substituted with one or two R₁₁; wherein each R₁₁ is independently selected from the group consisting of hydroxy and NR'R", and each of R' and R" is independently selected from the group consisting of hydrogen and C₁₋₆ alkyl;
R₂ is selected from the group consisting of hydrogen, halogen, cyano, C₁₋₈ alkyl, C₁₋₈ haloalkyl, C₁₋₈ alkoxy, and C₁₋₈ haloalkoxy; R₃ is selected from the group consisting of hydrogen, halogen, and C₁₋₈ alkyl; wherein at least one of R₂ and R₃ is not hydrogen;
each of R₄ and R₅ is independently selected from the group consisting of hydrogen, halogen, cyano, C₁₋₈ alkyl, C₁₋₈ haloalkyl, C₁₋₈ alkoxy, and C₁₋₈ haloalkoxy;
R₆ is selected from the group consisting of hydrogen, C₁₋₈ alkyl, C₁₋₈ haloalkyl, C₁₋₈ alkoxy, and C₁₋₈ haloalkoxy;
R₇ is selected from the group consisting of hydrogen, C₁₋₈ alkyl, C₁₋₈ haloalkyl, and C₁₋₈ alkoxy;
L is selected from the group consisting of -NR₈-, -O-, -S-, and -CR₉R₁₀-;
R₈ is selected from the group consisting of hydrogen, C₁₋₈ alkyl, C₁₋₈ haloalkyl, C₁₋₈ alkoxy, and C₁₋₈ haloalkoxy;
each of R₉ and R₁₀ is independently selected from the group consisting of hydrogen, hydroxy, C₁₋₈ alkyl, C₁₋₈ haloalkyl, C₁₋₈ alkoxy, and C₁₋₈ haloalkoxy; or R₉ and R₁₀, together with the C atoms to which they are attached, form a C₃₋₈ cyclic hydrocarbyl group optionally substituted with R₀, wherein R₀ is selected from the group consisting of halogen, hydroxy, C₁₋₃ alkyl, and C₁₋₃ alkoxy;
W is a 4- to 10-membered heterocyclyl optionally substituted with one or two R^{W}, the 4- to 10-membered heterocyclyl contains up to 3 heteroatoms independently selected from the group consisting of N and O, and the maximum number of O heteroatom is 1, wherein each R^{W} is independently selected from the group consisting of hydrogen, oxo, hydroxy, hydroxy C₁₋₈ alkyl, C₁₋₈ alkyl, C₁₋₈ haloalkyl, C₁₋₈ alkoxy, C₁₋₈ haloalkoxy, C₃₋₈ cycloalkyl, C₃₋₈ halocycloalkyl, and NR^{w1}R^{w2}; wherein each of R^{w1} and R^{w2} is independently selected from the group consisting of hydrogen and C₁₋₆ alkyl.

2. The compound according to claim 1, wherein R₁ is C₂₋₈ alkynyl or C₂₋₈ alkenyl.

3. The compound according to claim 1, wherein R₁ is ethynyl, ethenyl, or propynyl.

4. The compound according to claim 1, wherein R₄ and R₅ are hydrogen.

5. The compound according to claim 1, wherein one of R₂ and R₃ is C₁₋₃ alkyl, and the other is hydrogen.

6. The compound according to claim 1, wherein X₁ is CH.

7. The compound according to claim 1, wherein X₂ is -O-, -S-, -NH-, or -NC₁₋₃ alkyl-.

8. The compound according to claim 1, wherein X₂ is -O-.

9. The compound according to claim 1, wherein L is -NH-, -O-, -S-, or -CH₂-.

10. The compound according to claim 1, wherein L is -NH-.

11. The compound according to claim 1, wherein W is a 4- to 8-membered heterocyclyl optionally substituted with one or two R^{W}, the 4- to 8-membered heterocyclyl contains up to 3 heteroatoms independently selected from the group consisting of N and O, and the maximum number of O heteroatom is 1, wherein each R^{W} is independently selected from the group consisting of hydrogen, hydroxy, oxo, hydroxy C₁₋₆ alkyl, C₁₋₆ alkyl, C₁₋₆ haloalkyl, C₁₋₆ alkoxy, C₁₋₆ haloalkoxy, C₃₋₆ cycloalkyl, C₃₋₆ halo cyclic hydrocarbyl group, and NR^{w1}R^{w2}; wherein each of R^{w1} and R^{w2} is independently selected from the group consisting of hydrogen and C₁₋₆ alkyl.

12. The compound according to claim 1, wherein W is a 6-membered heterocyclyl containing a single N heteroatom optionally substituted with one or two R^{W}, wherein each R^{W} is independently selected from the group consisting of C₁₋₆ alkyl, hydroxy C₁₋₆ alkyl, and C₃₋₆ cycloalkyl.

13. The compound according to claim 1, wherein W is one of the following structural formulae: 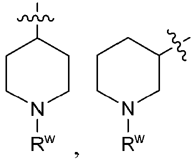 wherein each R^{W} is independently selected from the group consisting of C₁₋₃ alkyl, hydroxy C₁₋₃ alkyl, and C₃₋₆ cycloalkyl.

14. The compound according to claim 13, wherein W is one of the following structural formulae:

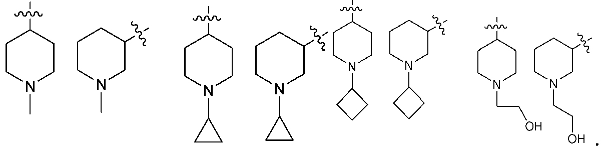

15. The compound according to claim 1, wherein the compound is selected from one of the following compounds:

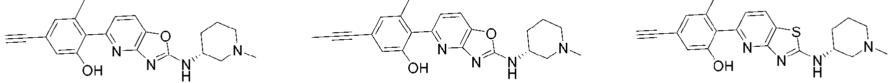

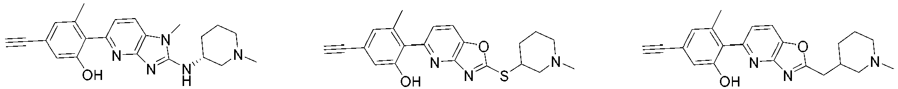

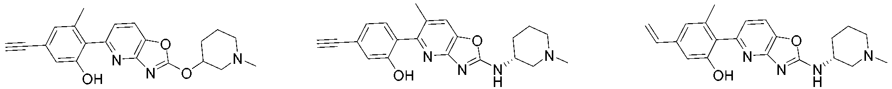

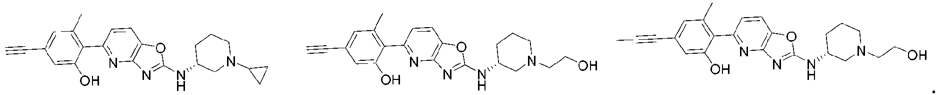

16. A pharmaceutical composition, comprising the compound according to any one of claims 1 to 15 and a pharmaceutically acceptable carrier, a diluent, or an excipient.

17. A method for treating or preventing an NLRP3-mediated disorder, comprising administering to a subject in need a therapeutically effective amount of the compound according to any one of claims 1 to 15 or the pharmaceutical composition according to claim 16.

18. The method according to claim 17, wherein the disorder is selected from the group consisting of autoimmune diseases, inflammatory disorders, cardiovascular diseases, neurodegenerative disorders, bacterial and viral infections, allergies, asthma, pancreatitis, multiple organ failure, kidney disease, platelet aggregation, cancer, transplantation, sperm motility, erythrocyte deficiency, transplant rejection, lung injury, respiratory diseases, and ischemic conditions.

19. The method according to claim 17 or 18, wherein the disorder is selected from the group consisting of Type II diabetes, atherosclerosis, Alzheimer's disease, aging, fatty liver, metabolic syndrome, asthma, psoriasis, obesity, acute and chronic tissue damage caused by infection, gout, arthritis, macular degeneration, enteritis, hepatitis, peritonitis, silicosis, UV-induced skin sunburn, contact hypersensitivity, sepsis, cancer, neurodegenerative diseases, multiple sclerosis, and Muckle-Wells syndrome.

20. The method according to claim 17, 18, or 19, wherein the NLRP3-mediated disorder is selected from the group consisting of rheumatoid arthritis, psoriatic arthritis, osteoarthritis, systemic lupus erythematosus, lupus nephritis, ankylosing spondylitis, osteoporosis, systemic sclerosis, multiple sclerosis, psoriasis, Type I diabetes, Type II diabetes, inflammatory bowel disease (Crohn's disease and ulcerative colitis), hyperimmunoglobulinemia D and periodic fever syndrome, cryopyrin-associated periodic syndromes, Schnitzler syndrome, systemic juvenile idiopathic arthritis, adult-onset Still's disease, gout, pseudogout, SAPHO syndrome, Castleman's disease, sepsis, stroke, atherosclerosis, celiac disease, DIRA (deficiency of IL-1 receptor antagonist), Alzheimer's disease, Parkinson's disease, and cancer.

21. Use of the compound according to any one of claims 1 to 15 in manufacture of a pharmaceutical for treating or preventing an NLRP3-mediated disorder.
